# EUROPEAN PATENT APPLICATION

(11) **EP 4 309 636 A1**
(43) Date of publication of application: **24.01.2024**
(21) Application number: 22837240.5
(22) Date of filing: 08.03.2022
(51) Int. Cl.: A61F 13/49, A61F 13/494, A61F 13/511, A61F 13/56

(54) **CONNECTION-TYPE DISPOSABLE WEARABLE ARTICLE**

(30) Priority: 07.07.2021 JP 2021112993
(71) Applicant: Daio Paper Corporation, Ehime 799-0492 (JP)
(72) Inventor: YAMASHITA, Yuichi, Shikokuchuo-shi, Ehime 799-0113 (JP)
(74) Representative: Meissner Bolte Partnerschaft mbB
(86) International application number: PCT/JP2022/010055
(87) International publication number: WO 2023/281818

(57) **Abstract**

The problem is to prevent leakage through between a waist end stretchable region and wearer's skin. The above problem is solved by that an end flap part EF, which extends backward so as to be located at a back side of an absorber 56, has a waist end stretchable region 79; the waist end stretchable region 79 has a pleat-suppressing region 90 in which a first sheet layer 71, a second sheet layer 72 bonded to an underside surface of the first sheet layer 71, and an elastic film 70 stacked in an underside of the second sheet layer 72 are overlapped; convex portions 31, which are swollen toward a top side away from the second sheet layer 72, are arranged in the first sheet layer 71 in a staggered arrangement; the first sheet layer 71 and the second sheet layer 72 are bonded to each other at bonded portions 80, each of which is formed between the convex portions 31 adjacent to each other in the width direction WD and between the convex portions 31 adjacent to each other in the front-back direction LD; a minimum width of gap portions 32, each of which is located between the convex portions 31 adjacent to each other in the width direction WD, is 0.1 to 0.9 times a maximum width of the convex portions 31, which are located at both front and back sides of the gap portion 32; and at least that portion of the second sheet layer 72 which is corresponding to a whole of the pleat-suppressing region 90 is bonded to the elastic film 70.

## Description

### Technical Field

The present invention relates to a connecting-type disposable wearing article such as a tape-type disposable diaper.

### Background Art

A general connecting-type disposable wearing article has a crotch portion including a center in a front-back direction, a ventral side part extending forward from the center in the front-back direction, and a dorsal side part extending backward from the center in the front-back direction, and at least the dorsal side part has wing parts extending both leftward and rightward so as to exceed a width of the crotch portion in a width direction. In addition, the wing parts have connecting portions that are detachably connected to an external surface of the ventral side part, and this external surface of the ventral side part has target portions to which the connecting portions are to be connected. During use, both the wing parts are turned from both sides of wearer's waist to the external surface of the ventral side part, and the connecting portions of the wing parts are connected to the target portions. Such a connecting-type disposable wearing article is used not only for babies and infants but also for nursing care (adult use) (for example, see Patent Literature 1).

A connecting-type disposable wearing article has generally poorer fitting in a lower torso direction than an underpants-type disposable wearing article. Thus, in particular, in order to improve prevention of leakage on wearer's dorsal side, it is proposed that a waist end stretchable region is provided along a width direction in a waist end portion of a dorsal side part (for example, see Patent Literature 1).

However, in a diaper according to Patent Literature 1, a large number of pleats continuous in a front-back direction are formed on a top surface of a waist end portion in a dorsal side part. Thus, even if a breathability is improved by clearances, each of which is formed between the pleats adjacent to each other, urine and muddy feces are likely to pass through between the pleats so as to be leaked outside.

### Citation List

### Patent Literature

Patent Literature 1: JP 2005-080859 A
Patent Literature 2: JP 2015-202251 A

### Summary of Invention

### Technical Problem

Therefore, a main object of the present invention is to prevent leakage through between a waist end stretchable region and wearer's skin.

### Solution to Problem

A connecting-type disposable wearing article solving the above mentioned problem is as follows.

### <First aspect>

A connecting-type disposable wearing article including:
a ventral side part extending forward from a center in a front-back direction, and a dorsal side part extending backward from the center in the front-back direction;
an absorber incorporated within a range from the ventral side part to the dorsal side part;
connecting portions, which are provided in both side portions of the dorsal side part and configured to be detachably connected to an external surface of the ventral side part; and
an end flap part, which extends backward so as to be located at a back side of the absorber,
wherein the end flap part includes
   a first sheet layer forming a skin-contact surface,
   a second sheet layer bonded to an underside surface of the first sheet layer, and
   an elastic film stacked in an underside of the second sheet layer,
the first sheet layer and the second sheet layer are made of nonwoven fabrics respectively,
a region including the elastic film has a waist end stretchable region, which can contract in a width direction and stretch in the width direction together with the elastic film,
the waist end stretchable region has a pleat-suppressing region, in which the first sheet layer, the second sheet layer and the elastic film are stacked,
at least in the pleat-suppressing region, convex portions, which are swollen toward a top side away from the second sheet layer, are provided in the first sheet layer at intervals in the front-back direction and in the width direction in a staggered arrangement, and the first sheet layer and the second sheet layer are bonded to each other at bonded portions, each of which is formed between the convex portions adjacent to each other in the width direction and between the convex portions adjacent to each other in the front-back direction,
a minimum width of gap portions, each of which is located between the convex portions adjacent to each other in the width direction, is 0.1 to 0.9 times a maximum width of the convex portions, which are located at both front and back sides of the gap portion, and
at least a portion of the second sheet layer which is corresponding to a whole of the pleat-suppressing region is bonded to the elastic film.

### (Effect)

On a top surface of the pleat-suppressing region of the present connecting-type disposable wearing article, little or no pleats extending linearly in the front-back direction are formed even in a natural length state, and this is especially true in a wearing state. More closely, when the waist end stretchable region stretches and contracts, in the pleat-suppressing region, although widths of the convex portions and those of the gap portions in the first sheet layer also stretch and contract, little or no pleats are formed since a whole of the first sheet layer does not deform wavily unlike a conventional wearing article. That is, since the convex portions, which are swollen toward the top side away from the second sheet layer, are previously provided in the first sheet layer at intervals in the staggered arrangement, to a certain level of stretching and contracting, the whole of the first sheet layer can stretch and contract in the width direction with little or no wavy deformation. As a result, even in the natural length state, little or no pleats are formed on the top surface of the pleat-suppressing region.

Meanwhile, a situation in which the convex portions are previously formed in the first sheet layer is similar to a situation in which pleats are previously formed thereon. Accordingly, there might be a possibility of leakage through portions between the convex portions. However, in the present invention, the convex portions are provided in the staggered arrangement and the minimum width of the gap portions, each of which is located between the convex portions adjacent to each other in the width direction, is 0.1 to 0.9 times the maximum width of the convex portions located at both the front and back sides of the gap portion. Accordingly, the portions between the convex portions are not continuous linearly along the front-back direction but continuous in a net-like (wavelike) way. In this way, excrement cannot move backward without colliding with the convex portions, that is, the convex portions become barriers to the movement of the excrement. Thus, prevention of the leakage is attained. On the other hand, the convex portions provided in the staggered arrangement decrease area of contact between the first sheet layer and wearer's skin, and in addition to this, the portions between the convex portions improve a breathability in a direction along a top surface of the first sheet layer.

Incidentally, in the pleat-suppressing region of the present connecting-type disposable wearing article, for example, as in Patent Literature 1 and Patent Literature 2, if bonding portions and non-bonding portions are provided alternately between the second sheet layer and the elastic film, when the pleat-suppressing region is contracted in the natural length state and in the wearing state, at the non-bonding portions, the second sheet layer is swollen away from the elastic film such that pleats extending linearly in the front-back direction would be formed. In this case, since the first sheet layer is integrated with the second sheet layer, the first sheet layer also deforms similarly such that pleats extending linearly in the front-back direction would be formed on the top surface of the first sheet layer (that is, the pleats cannot be suppressed). For this reason, it is necessary that at least that portion of the second sheet layer which is corresponding to the whole of the pleat-suppressing region is bonded to the elastic film.

Further, in addition to this, a fact that elasticity of the waist end stretchable region is generated not by an elongated elastic member such as a rubber thread but by the elastic film can be considered to have a positive influence for suppressing the pleats. That is, a contraction force of the elongated elastic member is concentrated linearly, but a contraction force of the elastic film is dispersed planarly. Accordingly, the contracting and stretching in the width direction of the second sheet layer is equalized entirely therein. This makes, also in the first sheet layer, the widths of the convex portions and the widths of the gap portions to be equalized. As a result, it is considered that the pleats are unlikely to be formed on the top surface of the first sheet layer.

### <Second aspect>

The connecting-type disposable wearing article according to the first aspect,
wherein a stretch rate of the waist end stretchable region in a spread state is 130 to 1700.

### (Effect)

In the above mentioned pleat-suppressing region, too large stretch rate makes it easy for the pleats to be formed (but it is still much difficult to form the pleats, comparing with conventional wearing articles) while too small stretch rate makes it meaningless to provide the stretchable region. Accordingly, the stretch rate of the waist end stretchable region is preferably within the range of the present aspect.

### <Third aspect>

The connecting-type disposable wearing article according to the first or second aspect,
wherein, a maximum width of each of the convex portions is 3 to 9 mm, and
in the pleat-suppressing region, rows, each of which includes 5 to 15 convex portions arranged in the front-back direction, are provided.

### (Effect)

A dimension of each of the convex portions and number of the convex portions can be determined as appropriate, but usually, they are preferably in the ranges of the present aspect, from viewpoints of an effect for suppressing the pleats and an effect for preventing the leakage.

### <Fourth aspect>

The connecting-type disposable wearing article according to any one of the first to third aspects,
wherein a height of each of the convex portions is 0.20 to 0.65 times a thickness of the absorber.

### (Effect)

In a disposable wearing article, a thickness of a portion including an absorber is largest. In this regard, a thickness of the end flap part is small because the end flap part includes no absorber. Thus, when the wearing article is worn, a clearance is likely to be formed between the end flap part and wearer's body surface. However, in the present aspect, since the height of each of the convex portions of the first sheet layer is enough with respect to the thickness of the absorber as a reference, a cushioning property (recoverability from a compressed situation in a thickness direction) of the end flap part is improved. As a result, a top surface of the end flap part becomes likely to be brought into contact with the wearer's body surface.

### <Fifth aspect>

The connecting-type disposable wearing article according to any one of the first to fourth aspects,
wherein the first sheet layer is made of a short fiber nonwoven fabric having a fineness of 1 to 3 dtex, a basis weight of 18 to 30 g/m², and a thickness of 0.3 to 1.4 mm, and
the second sheet layer is made of a short fiber nonwoven fabric having a fineness of 2 to 6 dtex, a basis weight of 17 to 30 g/m², and a thickness of 0.2 to 4 mm.

### (Effect)

A material of the first sheet layer and that of the second sheet layer can be determined as appropriate. However, it is preferable that the materials described in the present aspect are combined, because by doing so, shape maintainability of the convex portions of the first sheet layer becomes excellent, and in addition to this, the whole of the first sheet layer can stretch and contract easily in the width direction with little or no wavy deformation.

### <sixth aspect>

The connecting-type disposable wearing article according to any one of the first to fifth aspects, further including:
a wrapping sheet wrapping the absorber;
a liquid impervious sheet disposed in an underside level of that portion of the wrapping sheet which is located on an underside of the absorber; and
an exterior nonwoven sheet covering an underside surface of the liquid impervious sheet to constitute an external surface of the wearing article,
wherein the wrapping sheet extends to a middle of the end flap part in the front-back direction,
the first sheet layer, the second sheet layer, the liquid impervious sheet and the exterior nonwoven sheet extend over an entire length of the wearing article,
the pleat-suppressing region includes
   a first region having, between the first sheet layer and the exterior nonwoven sheet, only the second sheet layer, the wrapping sheet, the elastic film, the liquid impervious sheet and a hot melt adhesive for bonding them, and
   a second region having, between the first sheet layer and the exterior nonwoven sheet, only the second sheet layer, the elastic film, the liquid impervious sheet and the hot melt adhesive for bonding them, and
the elastic film extends from between the liquid impervious sheet and the portion of the wrapping sheet which is located on the underside level with respect to the absorber in the first region to between the second sheet layer and the liquid impervious sheet in the second region.

### (Effect)

As in the present aspect, in the pleat-suppressing region, stacked sheets other than the elastic film are preferably few in number, from a view point of reducing producing costs. However, only by decreasing the stacked number of sheets, stiffness of the pleat-suppressing region becomes lower and this region would be likely to bend at a boundary between the first region and the second region and at a tip end of the end flap part. On the other hand, in the present invention, the convex portions are formed in the first sheet layer, and in addition to this, the bonded portions, at which the first sheet layer and the second sheet layer are bonded to each other, are formed, as explained above. In this way, the stiffness of the pleat-suppressing region becomes higher comparing with a case in which there is no convex portion and no bonded portion, such that the pleat-suppressing region is unlikely to bend at the boundary between the first region and the second region and at the tip end of the end flap part.

### Advantageous Effects of Invention

The present invention provides advantages such as an ability to prevent leakage through between a waist end stretchable region and wearer's skin while a breathability is secured.

### Brief Description of Drawings

Fig. 1 is a plan view illustrating an internal surface of a tape-type disposable diaper in a state where the diaper is spread.
Fig. 2 is a plan view illustrating an external surface of the tape-type disposable diaper in the state where the diaper is spread.
Fig. 3 is a cross-sectional view taken along 3-3 line of Fig. 1.
Fig. 4 is a cross-sectional view taken along 4-4 line of Fig. 1.
Fig. 5 is a cross-sectional view taken along 5-5 line of Fig. 1.
Fig. 6 is a cross-sectional view taken along 6-6 line of Fig. 1.
Fig. 7 is a cross-sectional view taken along 7-7 line of Fig. 1.
Fig. 8 is a plan view illustrating a top sheet and an intermediate sheet in a spread state.
Fig. 9 is a plan view illustrating a main part of Fig. 8.
Fig. 10 is a plan view illustrating a main part of another example.
Fig. 11 is a plan view illustrating a main part of another example.
Fig. 12(a) is a cross-sectional view taken along 1-1 line of Fig. 11, Fig. 12(b) is a cross-sectional view taken along 2-2 line of Fig. 11, and Fig. 12(c) is a cross-sectional view taken along 9-9 line of Fig. 11.
Fig. 13 is a plan view illustrating a main part of an internal surface of a sample in a natural length state.
Fig. 14 is a plan view illustrating a main part of an external surface of the sample in the natural length state.
Fig. 15 is a plan view illustrating a main part of an internal surface of a commercially available product in a natural length state.
Fig. 16 is a plan view illustrating a main part of an external surface of the commercially available product in the natural length state.

### Description of Embodiments

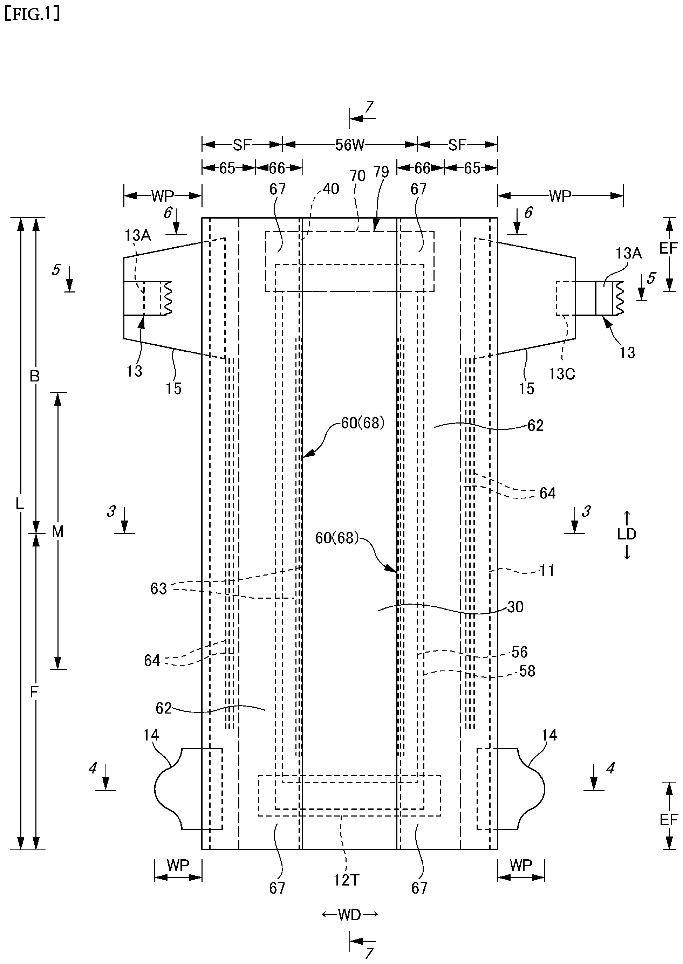

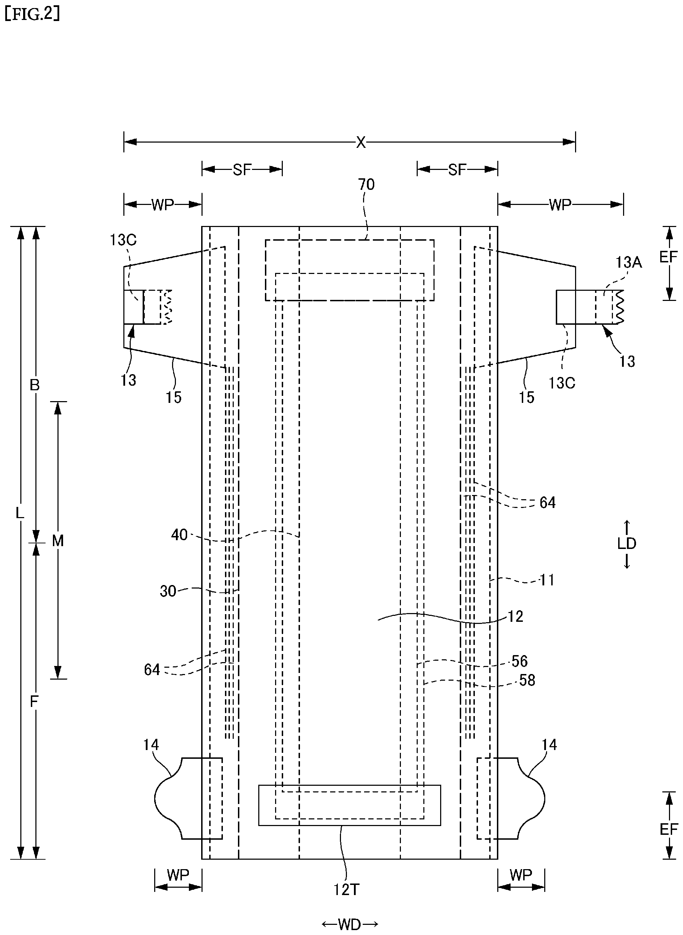

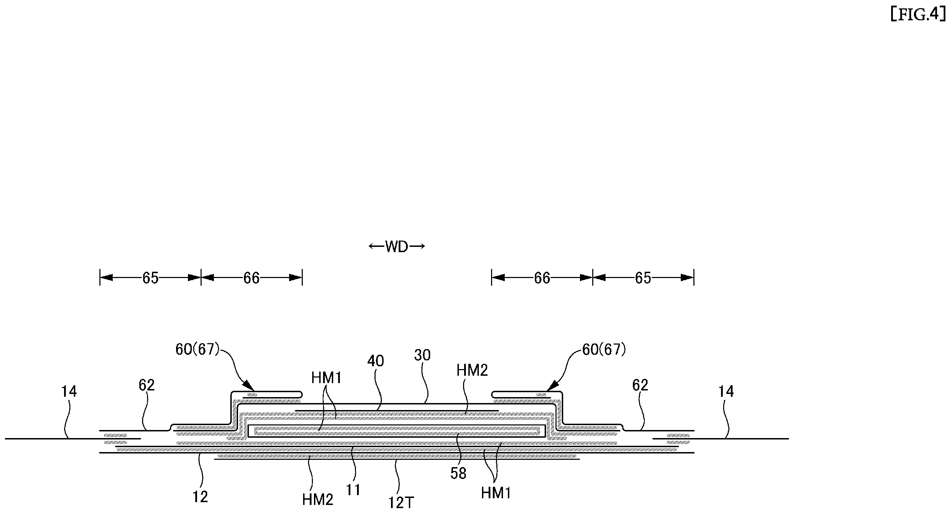

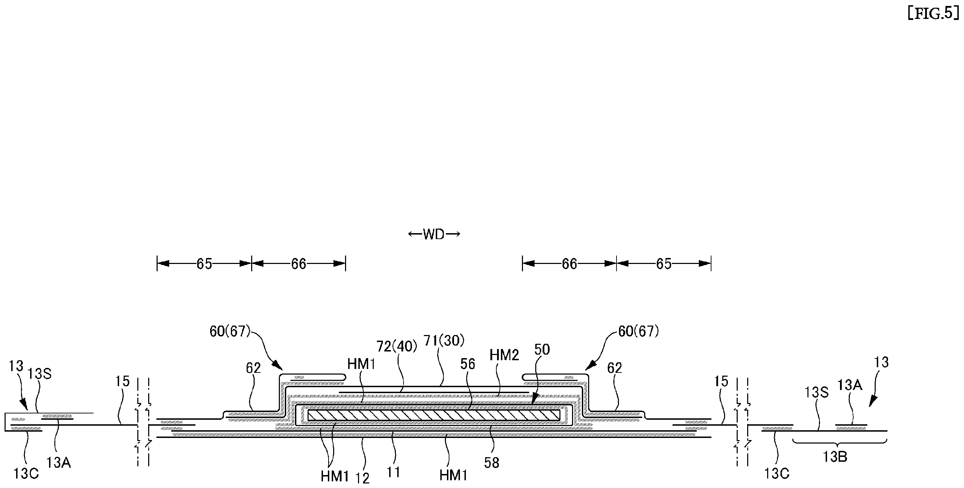

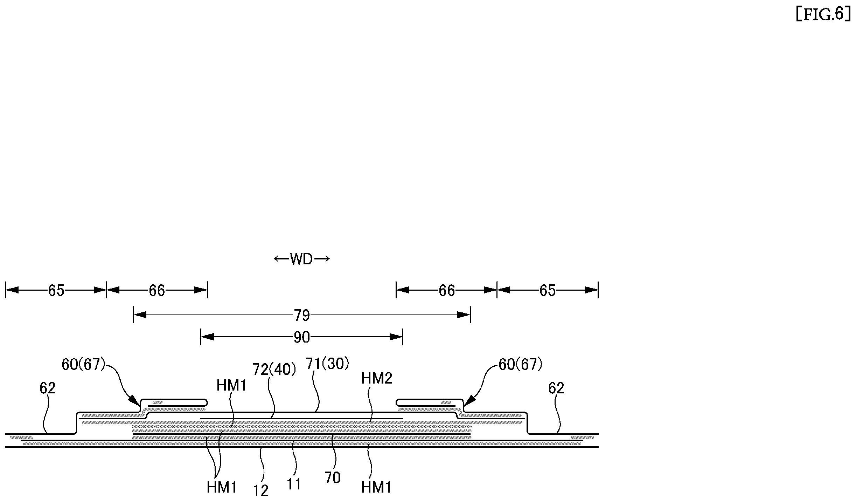

Figs. 1 to 7 show an example of a tape-type disposable diaper. In the figures, reference character X refers to a maximum width of the diaper exclusive of connecting tapes 13, whereas reference character L refers to a maximum length of the diaper. In the sectional views, each dotted pattern region represents a hot melt adhesive as a joining means for bonding constituent members to each other, which are located in a top side and an underside of the dotted pattern. The hot melt adhesive can be applied by a known method such as slot application, bead application into a continuous line or a dot line, spray application into a spiral shape, a Z shape, etc., or by pattern coating (transfer of a hot melt adhesive by a letterpress method). Alternatively, in the fixed portion of an elastic member, instead of or in addition to the above applications of the hot melt adhesive, the hot melt adhesive is applied to an outer peripheral surface of the elastic member, and the elastic member can be fixed to a member located adjacent thereto. Examples of the hot melt adhesive include an EVA-based agent, a pressure sensitive adhesive rubber-based agent (elastomer-based agent), a polyolefin-based agent, and a polyester/polyamide-based agent, and these can be used without particular limitation. As a bonding means for bonding the respective constituent members to each other, a means by material welding such as heat sealing or ultrasonic sealing also can be used.

The present tape-type disposable diaper has a ventral side part F extending forward from a center in a front-back direction LD and a dorsal side part B extending backward from the center in the front-back direction LD. Reference character M refers to a crotch portion defined as a range including the center in the front-back direction LD. If an absorber 56 has a narrower portion in a middle in the front-back direction, the crotch portion M refers to a range in the front-back direction LD having the narrower portion. Then, if the absorber 56 does not have the narrower portion, and instead, as in the illustrated example, an outer shape of the product in a spread state has a narrowed portion, the crotch portion M refers to a range in the front-back direction LD having the narrowed portion of the outer shape. Further, if the absorber 56 has neither of the narrower portion and the narrowed portion, the crotch portion M refers to a portion positioned at a middle when a product is divided into equal thirds in the front-back direction LD. Further, the present tape-type disposable diaper includes the absorber 56 incorporated within a range from the ventral side part F to the dorsal side part B, a liquid-pervious top sheet 30 covering a top side of the absorber 56, a liquid-impervious sheet 11 covering an underside of the absorber 56, and an exterior nonwoven sheet 12 covering an underside of the liquid-impervious sheet 11 to constitute an external surface of the product.

Hereinafter, a material and a characteristic part of each portion will be described in due order.

### (Absorber)

The absorber 56 can have a shape having the narrower portion along peripheries of wearer's legs in a middle in the front-back direction (like an hourglass shape) in addition to a rectangular shape as in the illustrated example. Reference character 56x indicates a maximum width of the absorber 56. The absorber 56 is a portion, which absorbs and holds excrement liquid and may be formed by an assembly of fibers. As this fiber assembly, in addition to one obtained by accumulating short fibers such as fluff pulps or synthetic fibers, a filament assembly obtained by opening a tow (fiber bundle) of synthetic fibers such as cellulose acetate as necessary can also be used. In a case where fluff pulps or short fibers are accumulated, a fiber basis weight may be, for example, about 100 to 300 g/m². In a case of a filament assembly, a fiber basis weight may be, for example, about 30 to 120 g/m². In a case of a synthetic fiber, a fineness is, for example, 1 to 16 dtex, preferably 1 to 10 dtex, and more preferably 1 to 5 dtex. In a case of a filament assembly, as a filament, it is possible to use a non-crimped fiber but it is preferable to use a crimped fiber.

### (Super Absorbent Polymer Particles)

The absorber 56 may partially or entirely contain super absorbent polymer particles. The super absorbent polymer particles include "powder" in addition to "particles". As the super absorbent polymer particles, those used for this type of absorbent article can be used as they are. The particle diameters of the super absorbent polymer particles are not particularly limited. However, for example, when sieving using a standard sieve of 500 µm (JIS Z8801-1: 2006) (shake for five minutes) is performed, and particles falling under the sieve using this sieving are sieved using a standard sieve of 180 µm (JIS Z8801-1: 2006) (shake for five minutes), it is desirable that a ratio of particles remaining on the standard sieve of 500 µm is 30% by weight or less, and a ratio of particles remaining on the standard sieve of 180 µm is 60% by weight or more.

A material of the super absorbent polymer particles can be used without particular limitation, but those having a water absorption capacity of 40 g/g or more are preferable. Examples of the super absorbent polymer particles include a starch-based material, a cellulose-based material, and a synthetic polymer-based material. A starch-acrylic acid (salt) graft copolymer, a saponified product of a starch-acrylonitrile copolymer, a cross-linked product of sodium carboxymethyl cellulose, an acrylic acid (salt) polymer, or the like can be used. As the shapes of the super absorbent polymer particles, a usually used particulate material shape is suitable, but other shapes also can be used.

As the super absorbent polymer particles, those having a water absorption rate of 70 seconds or less, particularly of 40 seconds or less are suitably used. When the water absorption rate is too slow, so-called returning that a liquid supplied into the absorber 56 returns out of the absorber 56 tends to occur.

As the super absorbent polymer particles, those having a gel strength of 1000 Pa or more are suitably used. This makes it possible to effectively suppress feeling of stickiness after liquid absorption even in a case of using a bulky absorber 56.

The basis weight of the super absorbent polymer particles can be appropriately determined depending on the absorption amount required for the usage of the absorber 56. Thus, although it is not necessarily appropriate to suggest, the basis weight may be, in a usual case, within the range of 50 to 350 g/m².

### (Wrapping Sheet)

In order to prevent escape of the super absorbent polymer particles or to improve shape maintainability of the absorber 56, the absorber 56 can be incorporated in the form of an absorbent element 50 in which the absorber 56 is wrapped by a wrapping sheet 58. As the wrapping sheet 58, tissue paper, particularly crepe paper, a nonwoven fabric, a polyethylene laminated nonwoven fabric, a perforated sheet, or the like can be used. However, it is desirable that the wrapping sheet 58 is a sheet from which super absorbent polymer particles do not escape. When a nonwoven fabric is used instead of crepe paper, a hydrophilic spunbond/meltblown/meltblown/spunbond (SMMS) nonwoven fabric is particularly suitable, and polypropylene, polyethylene/polypropylene, or the like can be used as a material thereof. A nonwoven fabric having a fiber basis weight of 5 to 40 g/m², particularly of 10 to 30 g/m² is desirable.

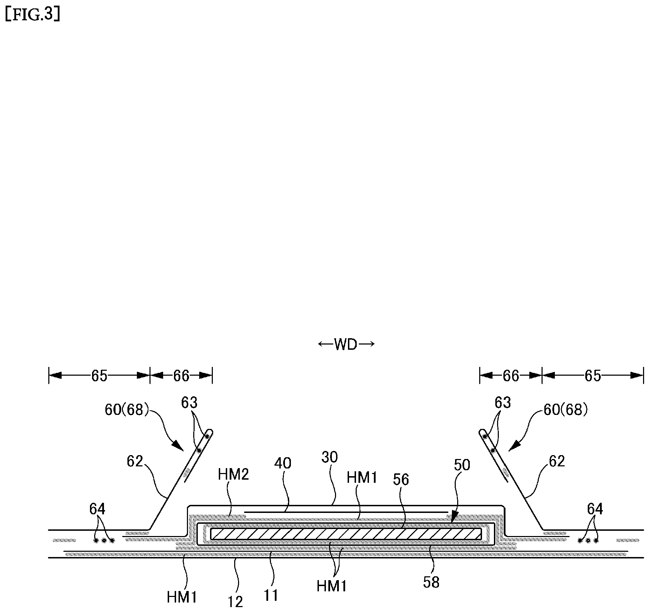

As illustrated in Fig. 3, the single wrapping sheet 58 may wrap the whole of the absorber 56. In addition to this wrapping manner, a plurality of the wrapping sheets 58 such as upper and lower two wrapping sheets may wrap the whole of the absorber 56. The wrapping sheet 58 can be omitted.

### (Top Sheet)

As the top sheet 30 having liquid perviousness, a perforated or non-perforated nonwoven fabric, a porous plastic sheet, etc. can be used.

The top sheet 30 extends from a front end to a back end of the product in the front-back direction LD and extends to the lateral sides beyond the absorber 56 in a width direction WD. However, for example, when lines, from which rising gathers 60 described later start to rise, are located so as to be closer to the center in the width direction WD than side edges of the absorber 56, appropriate deformation can be made, for example, a width of the top sheet 30 is made shorter than the maximum width of the absorber 56 as necessary.

### (Intermediate Sheet)

For preventing returning of a liquid that has passed through the top sheet 30, an intermediate sheet 40 can be provided so as to be adjacent to an underside of the top sheet 30. In a case where the intermediate sheet 40 is not used for forming a second sheet layer 72 discussed later, the intermediate sheet 40 can also be omitted.

As the intermediate sheet 40, various kinds of nonwoven fabrics can be used, and particularly, a bulky air-through nonwoven fabric can be preferably used. As the air-through nonwoven fabric, a composite fiber having a core-sheath structure is preferably used. In this case, a resin used for the core may be polypropylene (PP) but is preferably polyester (PET) having high rigidity. A basis weight is preferably 17 to 80 g/m², more preferably 25 to 60 g/m². A fineness of a raw material fiber of the nonwoven fabric is preferably 2.0 to 10 dtex. In order to increase the bulkiness of the nonwoven fabric, it is preferable to use, totally or partly for the raw material fiber, an eccentric fiber having no core in a center, a hollow fiber, and an eccentric and hollow fiber as a mixed fiber.

The intermediate sheet 40 in the illustrated example is disposed at the center in the width direction so as to be shorter than the width of the absorber 56, but may be disposed over the maximum width. The intermediate sheet 40 may be disposed over the maximum length of the diaper, but may be disposed only in a middle portion in the front-back direction LD including an excrement position as in the illustrated example.

### (Liquid Impervious Sheet)

Although the liquid impervious sheet 11 is not particularly limited, it has preferably moisture perviousness. As the liquid impervious sheet 11, for example, a microporous sheet can be used preferably which is obtained by kneading an inorganic filler in a polyolefin-based resin such as polyethylene or polypropylene, molding the kneaded mixture into a sheet, and stretching the sheet in one or two axial directions. Furthermore, the liquid impervious sheet 11 can be used which has a nonwoven fabric as a base material to enhance waterproof property.

It is desirable that the liquid impervious sheet 11 extends over the same range as or a wider range than that of the absorber 56 in the front-back direction LD and the width direction WD. However, for example, when another water blocking means is present, as another possible configuration, the end portion of the absorber 56 does not have to be covered with the liquid impervious sheet 11 in the front-back direction LD or the width direction WD as necessary.

### (Exterior Nonwoven Sheet)

The exterior nonwoven sheet 12 covers a whole of an underside of the liquid impervious sheet 11 and imparts a cloth-like appearance to the external surface of the product. In addition to using a single piece of nonwoven fabric, it is also possible to use stacked multiple nonwoven fabrics. In the latter case, the nonwoven fabrics are preferably bonded to each other with a hot melt adhesive or the like. When the nonwoven fabric is used, it is preferable that a constituent fiber of the nonwoven fabric has a fineness of 1.6 to 2.3 dtex, and the nonwoven fabric has a basis weight of 15 to 25 g/m² and a thickness of 0.3 to 0.8 mm.

### (Rising Gather)

In order to prevent so-called side leakage by blocking movement of the excrement transmitted laterally on the top sheet 30, in both side portions on a top surface of the top sheet 30 in the width direction WD, the rising gathers 60 rising from the top surface of the top sheet 30 are provided along blocking positions against the excrement.

More specifically, each of the rising gathers 60 has: a root portion 65 fixed in a region including a side flap part SF, a main portion 66 extending from the root portion 65, a front fallen portion 67f and a back fallen portion 67b formed by fixing a front end portion and a back end portion of the main portion 66 in a fallen state respectively, a rising portion 68 formed to be non-fixed between the front fallen portion 67f and the back fallen portion 67b in the main portion 66, and in addition, a gather elastic member 63 is attached at least to a tip portion of the rising portion 68.

Each member of the rising gather 60 in the illustrated example is formed of a gather sheet 62. This gather sheet 62 is two-folded at a tip (an end being opposite to the root portion 65) of the main portion 66 to form a two-layered structure. The gather elastic member 63 is interposed between two layers of this two-layered structure. The gather elastic member 63 may be provided only in the rising portion 68. However, it is preferable that, as in the illustrated example, the gather elastic member 63 is fixed in a range from a back end portion of the front fallen portion 67f to a front end portion of the back fallen portion 67b, because a contraction force of the gather elastic member 63 is acted not only over a whole of the rising portion 68 but also over a range including the back end portion of the front fallen portion 67f and the front end portion of the back fallen portion 67b.

An internal surface of the gather sheet 62 has a fixed start point in the width direction WD on a side portion of the top sheet 30. That portion of the gather sheet 62 which is located at an outer side in the width direction is bonded by a hot melt adhesive or the like to an internal surface of the side flap part SF, namely in the illustrated example, a side portion of the liquid impervious sheet 11, and a side portion of the exterior nonwoven sheet 12, which is located at an outer side in the width direction of the side portion of the liquid impervious sheet 11.

That portion of the each of the rising gathers 60 which is located at an inside in the width direction of the fixed start point, is fixed to the top sheet 30 at both end portions in the front-back direction. However, the rising portion 68 therebetween is the non-fixed free portion. Accordingly, the rising portion 68 rises due to the contraction force of the gather elastic member 63. Therefore, the first rising portion 68 fits closely to wearer's body surface. In addition, the rising portion 68 is contracted in the front-back direction due to the contraction force of the gather elastic member 63, a portion including the front fallen portion 67f and a portion including the back fallen portion 67b are deformed so as to close to each other.

Although not illustrated, as is well known, both end portions of the main portion 66 of the rising gather 60 can be fixed as the fallen portions in a two-folded state having a base edge side portion extending inward in the width direction from an outer side portion in the width direction, and a tip side portion folded back to a body side from an end edge of the base edge side portion on a central side in the width direction and extending outward in the width direction.

A kind of the gather sheet 62 is not particularly limited. However usually, in order to secure a liquid barrier property, a water-repellent sheet is used. In particular, a nonwoven fabric having a meltblown layer sandwiched between spunbond layers (an SMS nonwoven fabric, an SMMS nonwoven fabric, an SSMS nonwoven fabric, or an SSMMS nonwoven fabric) is suitable from a viewpoint of achieving both the texture and the liquid barrier property. In addition to using a single piece of nonwoven fabric, it is also possible to use stacked multiple nonwoven fabrics. In the latter case, the nonwoven fabrics are preferably bonded to each other with a hot melt adhesive or the like.

As the gather elastic member 63, a rubber thread (spandex rubber thread having the fineness of about 420 to 1120 dtex) can be used. As illustrated in Fig. 1 and Fig. 3, a plurality of the gather elastic member 63 may be provided on each side, or one gather elastic member 63 may be provided on each side. Further, a stretch rate of the gather elastic member 63 in the spread state can be appropriately determined, but for example, it is possible to be about 230 to 320%.

### (Side flap part)

The tape-type disposable diaper in the illustrated example has a pair of the side flap parts SF without the absorber 56 on lateral sides of both the side edges of the absorber 56, respectively. As in the illustrated example, each of the side flap parts SF can be formed of a material which is continuous from a portion including the absorber 56 (the exterior nonwoven sheet 12 or the like). Alternately, the side flap part SF can be formed by attaching another material to the portion including the absorber 56.

### (Plane Gather)

To the each of the side flap parts SF, a side elastic member 64, which is an elongated elastic member formed of rubber thread or the like, is fixed in a stretched state along the front-back direction LD, hence an around-leg portion in the side flap part SF is configured as a plane gather. The side elastic member 64 can be provided between the liquid impervious sheet 11 and the exterior nonwoven sheet 12 in the side flap part SF, in addition to between the gather sheet 62 and the liquid impervious sheet 11 in an outer side in the width direction in the vicinity of the fixed start point in a bonded section of the gather sheet 62 as in the illustrated example. A plurality of the side elastic members 64 may be provided on each side, as illustrated in Fig. 1 and Fig. 3, or only one side elastic member 64 may be provided on each side.

The plane gather is a portion to which a contraction force of the side elastic member 64 is applied (a portion in which the side elastic members 64 are illustrated in the drawings). Thus, in addition to a configuration in which the side elastic members 64 are present only in a site of the plane gather, there may be other configurations. In each of these other configurations, the side elastic members 64 are present in a range from the plane gather to a front side, to a back side, or to both the front and back sides of the plane gather. However, in other sites than the plane gather, the side elastic members 64 are finely cut at one place or at many places, the side elastic members 64 are not fixed to sheets sandwiching the side elastic members 64, or both the above two measures are carried out. As a result, the contraction force is not applied to the other sites than the plane gather (which is substantially equivalent to that the elastic members are not provided on the other sites) such that the contraction force of the side elastic members 64 is applied only to the site of the plane gather.

### (Wing part)

In the present tape-type disposable diaper, the dorsal side part B has wing parts WP extending outward so as to exceed a width of the crotch portion M in the width direction WD. Similarly, the ventral side part F has also wing parts WP extending outward so as to exceed a width of the crotch portion M in the width direction WD. These wing parts WP can be formed by sheet shaped members 14, 15 different from the other parts. Further, although not illustrated, in a structure having the side flap parts SF, when a middle in the front-back direction LD in each of side portions of the side flap parts SF is cut to form a concave edge from a side edge of the crotch portion M to a lower edge of the wing part, and as a result the wing part WP is formed.

In a case where, as in the illustrated example, the wing part WP in the dorsal side part B is formed by the sheet shaped member 15 different from the other parts, as this sheet shaped member 15, an elastic film can be used, whose both top and underside surfaces are covered with fiber-stacking layers or nonwoven fabric layers, and which can stretch and contract in the width direction WD. In this case, it is preferable that stress required for stretching the sheet shaped member 15 of the wing part WP is set to be larger than stress for stretching a waist end stretchable region 79 discussed later.

### (Connecting Portion)

In the illustrated example, the connecting tapes 13 are provided in the wing parts WP in the dorsal side part B respectively. Then, connecting portions 13A configured to be detachably connected to an external surface of the ventral side part F are provided on the connecting tapes 13. When the diaper is worn, the connecting tapes 13 are turned from both sides of wearer's waist to the external surface of the ventral side part F, and the connecting portions 13A of the connecting tapes 13 can be connected to appropriate connection sites on the external surface of the ventral side part F. Although not illustrated, in the dorsal side part B, it is also possible to make a dimension of the wing part WP in the width direction WD sufficiently large in order to attach directly the connecting portion 13A to internal surface of the wing part WP.

As illustrated in Fig. 1, Fig. 2 and Fig. 5, each of the connecting tapes 13 has a sheet 13S and a connecting portion 13A for the ventral side part F. The sheet 13S is composed of a base end portion 13C fixed to the wing part WP and a main unit portion 13B protruded from the base end portion 13C. The connecting portion 13A is provided at an intermediate portion of the main unit portion 13B in the width direction WD in the sheet 13S. In this main unit portion 13B, a portion located closer to a base end portion 13C than the connecting portion 13A refers to a non-connecting portion unconnected to the ventral side part F, whereas a portion on an opposite side to this non-connecting portion with respect to the connecting portion 13A refers to a tab portion. The non-connecting portion and the tab portion are formed only of that portion of the sheet 13S which forms the main unit portion 13B.

The connecting portion 13A is formed of a hook material (male member) of a mechanical fastener (hook and loop fastener). The hook material has many engaging projections on a connecting surface thereof. Examples of the shapes of the engaging projections include a tick shape, a J shape, a mushroom shape, a T shape, and a double J shape (a shape in which the J-shaped ones are connected to each other back to back), but any shape may be used.

As the sheet 13S composed of the base end portion 13C and the main unit portion 13B, a nonwoven fabric, a plastic film, a polyethylene laminated nonwoven fabric, paper, or a composite material thereof can be used.

The connecting portion 13A, in the illustrated example, is provided on the sheet 13S of the connecting tape 13 protruded from the wing part WP. However, the connecting portion 13A may be provided directly on the wing part WP.

### (Target Sheet)

A target sheet 12T is provided on the connection sites on the ventral side part F to be connected to the connection sites.

A sheet material for forming the target sheet 12T is not particularly limited. However, when the connecting portion 13A is a hook material, for example, it is possible to use a long fiber nonwoven fabric in which fibers are partially welded to each other by ultrasonic welding of an intermittent pattern. In this case, the long fiber nonwoven fabric is preferably a nonwoven fabric in which a constituent fiber has a fineness of 5 to 10 dtex, a basis weight of 25 to 40 g/m², and a thickness of 0.3 to 0.8 mm.

Further, when the connecting portion 13A is the hook material, as the target sheet 12T, it is also possible to use a sheet material in which many loop yarns are provided on a surface of a base material made of a plastic film or a nonwoven fabric and the engaging projections of the hook material are entangled with the loop yarns. A specific example of such sheet material used for the target sheet 12T is a composite sheet material in which loop pile fiber yarns are woven to be appeared on at least an external surface of a base material. In this composite sheet material, on the external surface of the base material, that is, on an external surface side of the disposable diaper, the loop pile fiber yarns are formed to protrude at intervals in a weft direction and a warp direction, whereas on an underside (wearer's side) of the base material, the pile fiber yarns are combined with each other to form intersection rows of pile fiber warps.

Further, when the connecting portion 13A is the hook material, on the ventral side part F, the connection site to which the connecting tape 13 is connected can be made of a nonwoven fabric (for example, the exterior nonwoven sheet 12 is provided). In this instance, it is possible to dispose, on an inside of the exterior nonwoven sheet 12, the target sheet 12T made of a plastic sheet, paper, a nonwoven fabric, etc. on which a connecting position can be indicated by, for example, using a printed scale or the like. In this case, the hook material of the connecting portion 13A is entangled with fibers of the exterior nonwoven sheet 12 at the position of the target sheet 12T which can be seen by a user through the exterior nonwoven sheet 12a such that the user can perform the connecting.

On the other hand, in a case where the connecting portion 13A is an adhesive layer, it is possible to use, as the target sheet 12T, a sheet material made of a plastic film having a smooth surface, which is rich in adhesiveness, and subjected to a peeling process.

### (End Flap Part)

The present tape-type disposable diaper has a pair of end flap parts EF on a front side and a back side of the absorber 56, respectively, which do not include the absorber 56. The constituent members of each of the end flap parts EF change depending on the structure of the diaper. For example, the end flap part EF is formed by stacking a portion of the top sheet 30, a portion of the intermediate sheet 40, a portion of the wrapping sheet 58, a portion of the gather sheet 62, a portion of the liquid impervious sheet 11 and a portion of the exterior nonwoven sheet 12, which extend to the front side or the back side of the absorber 56 to be bonded to one another. Unlike the illustrated example, in a case where the exterior nonwoven sheet 12 is not included in a diaper, for example, the end flap part EF can be formed with the top sheet 30, the intermediate sheet 40, the wrapping sheet 58 and the liquid impervious sheet 11. The end flap part EF may be formed by adding a sheet, which is dedicated for forming the end flap part EF, to the front side or the back side of the absorber 56.

It is preferable that a dimension in the front-back direction LD of the end flap part EF in the dorsal side part B is longer than a dimension in the front-back direction LD of the base end portion 13C in the connecting tape 13. Usually, the dimension in the front-back direction LD of the end flap part EF is preferably about 20 to 250 of the dimension L in the front-back direction LD of the whole of the diaper. As an example, the dimension in the front-back direction LD of the end flap part EF of a diaper for babies and infants can be set to be about 80 to 120 mm.

### (Waist End Stretchable Region)

As illustrated in Fig. 1 and Fig. 6 to Fig. 8, the end flap part EF in the dorsal side part B includes a first sheet layer 71 forming a skin-contact surface, a second sheet layer 72 bonded to an underside surface of the first sheet layer 71, and an elastic film 70 stacked in an underside of the second sheet layer 72. Further, a region including the elastic film 70 contracts in the width direction WD together with the elastic film 70, and has the waist end stretchable region 79, which can stretch in the width direction WD. A whole of the region having the elastic film 70 may refer to the waist end stretchable region 79. Alternatively, only a part of the region having the elastic film 70 may refer to the waist end stretchable region 79, that is, some parts of the region having the elastic film 70 (e.g., parts located at both end portions of this region in the width direction WD) have little or no elasticity.

As long as the waist end stretchable region 79 is provided in the end flap part EF in the dorsal side part B, a dimension and an arrangement of the waist end stretchable region 79 may be determined as appropriate. A position of a front end of the waist end stretchable region 79 may be the same as a position of a back end of the absorber 56. Alternatively, the front end of the waist end stretchable region 79 may be located between the back end of the absorber 56 and a back end of the wrapping sheet 58, may be located between the back end of the wrapping sheet 58 and a back end of the product (a waist edge in the back side), or on the contrary, may be located at a front side of the back end of the absorber 56. In addition, a position of a back end of the waist end stretchable region 79 may be the same as a position of the back end of the product (a waist edge in the back side), or may be about 10 to 20 mm away therefrom on the front side.

It is desirable that a width of the waist end stretchable region 79 is substantially the same as or more than the width of the absorber 56, but not limited thereto. For example, the waist end stretchable region 79 preferably extends to outer sides beyond positions, each of which is 10 mm apart from the side edge of the absorber 56 on a center side in the width direction WD. Therefore, although not shown, the waist end stretchable region 79 may extend to be close to the wing parts WP, and may extend to be entered into the wing parts WP.

A stretch rate of the waist end stretchable region 79 in the spread state can be appropriately determined, and for example, it is possible to be about 120 to 220%.

The elastic film 70 is not particularly limited, and as long as it has elasticity in itself, any film can be used without specific limitation, which is obtained by, for example, processing one kind of thermoplastic elastomer or a blend of more than one kind of thermoplastic elastomers, e.g., a styrene-based elastomer, a polyolefin-based elastomer, a polyamide-based elastomer, a polyurethane-based elastomer, etc., is processed, through extrusion such as a T-die method and a inflation method, so as to be film shaped. As the elastic film 70, in addition to a nonporous sheet, it is possible to use a sheet in which a plurality of holes or slits is formed for a breathability. In particular, it is preferable that the elastic film 70 has a tensile strength in the stretchable direction of 8 to 25 N/35 mm, a tensile strength in a direction orthogonal to the stretchable direction of 5 to 20 N/35 mm, a tensile elongation in the stretchable direction of 450 to 1,0500, and a tensile elongation in the direction orthogonal to the stretchable direction of 450 to 1,4000. The tensile strength and the tensile elongation (breaking elongation) refer to values measured by setting an initial chuck interval to 50 mm and a tensile speed to 300 mm/min in accordance with JIS K7127: 1999 "Plastics -Determination of tensile properties-" except that the test piece has a rectangular shape of width 35 mm × length 80 mm. As a tensile testing machine, for example, AUTOGRAPH AGS-G100N manufactured by SHIMADZU CORPORATION can be used. A thickness of the elastic film 70 is not particularly limited. However, the thickness is preferably about 20 to 40 um. A basis weight of the elastic film 70 is not particularly limited. However, the basis weight is preferably about 30 to 45 g/m² and more preferably about 30 to 35 g/m².

As long as the first sheet layer 71 and the second sheet layer 72 are made of nonwoven fabrics, they are not particularly limited and for example, they can be formed by constituent members of the end flap part EF. That is, as in the illustrated example, the first sheet layer 71 can be formed by that portion of the top sheet 30 which is located in the end flap part EF, whereas the second sheet layer 72 can be formed by that portion of the intermediate sheet 40 which is located in the end flap part EF. It is needless to say that the first sheet layer 71 and the second sheet layer 72 may be formed by adding sheets, which are different from the top sheet 30 and the intermediate sheet 40 and which are dedicated for forming the first sheet layer 71 and the second sheet layer 72, respectively. Alternatively, for the first sheet layer 71 and the second sheet layer 72, it is possible that either one of the two layers is formed by such a separate sheet dedicated for forming it whereas the other of the two layers is formed by one of the constituent members of the end flap part EF. Further, the first sheet layer 71 and the second sheet layer 72 may be formed of two separate sheets, or, although not illustrated, a single sheet may be two-folded such that with respect to a fold line of the single sheet, the first sheet layer 71 refers to a portion thereof on one side and the second sheet layer 72 refers to a portion thereof on the other side.

A material of the first sheet layer 71 and that of the second sheet layer 72 can be determined as appropriate. However it is preferable that the first sheet layer 71 is made of a short fiber nonwoven fabric having a fineness of 1 to 3 dtex, a basis weight of 18 to 30 g/m², and a thickness of 0.3 to 1.4 mm. Further, the second sheet layer 72 is made of a short fiber nonwoven fabric having a fineness of 2 to 6 dtex, a basis weight of 17 to 30 g/m², and a thickness of 0.2 to 4 mm. By combining these materials, shape maintainability of convex portions 31 discussed later of the first sheet layer 71 becomes excellent, and in addition to this, the whole of the first sheet layer 71 can stretch and contract in the width direction WD with little or no wavy deformation.

As long as the first sheet layer 71 and the second sheet layer 72 are provided over a whole of a pleat-suppressing region 90 discussed later, these sheet layers 71 and 72 may extend over a wider range than that of the waist end stretchable region 79, may extend over the same range as that of the waist end stretchable region 79, or may extend so as to locate inside of the peripheral edge of the waist end stretchable region 79 at least in one of the front-back direction LD and the width direction WD. For example, as in an illustrated example, the following configuration is possible. That is, in the front-back direction LD, the top sheet 30 forming the first sheet layer 71 and the intermediate sheet 40 forming the second sheet layer 72 extend to both a front side and a back side of the waist end stretchable region 79, and in the width direction WD, the top sheet 30 forming the first sheet layer 71 extends to both outer sides of the waist end stretchable region 79, whereas both side edges of the intermediate sheet 40 forming the second sheet layer 72 may be located so as to be closer to the center than both side edges of the waist end stretchable region 79.

### (Pleat-Suppressing Region)

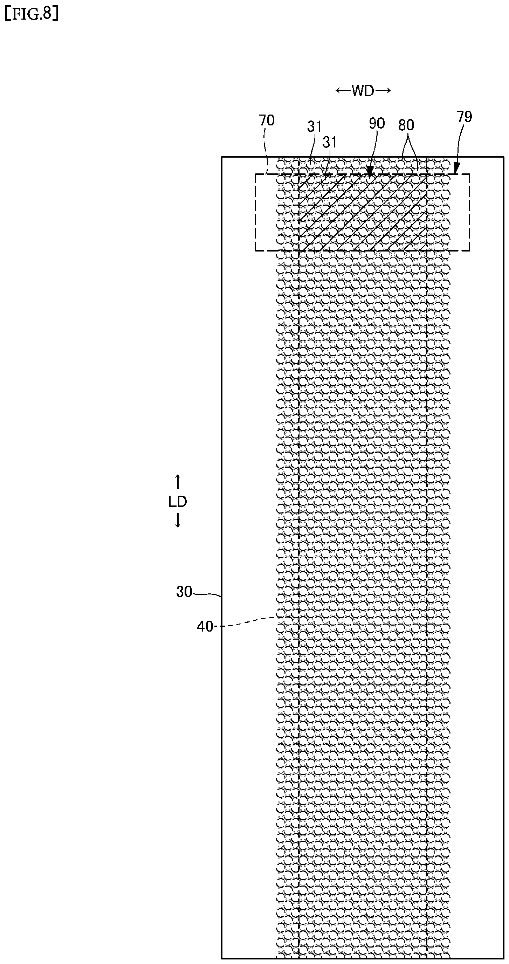

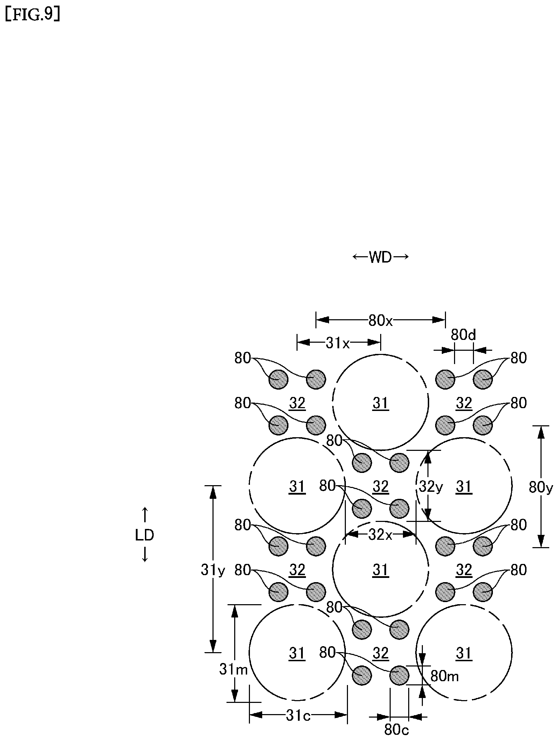

The waist end stretchable region 79 has the pleat-suppressing region 90 in which the first sheet layer 71, the second sheet layer 72 and the elastic film 70 are stacked. In the pleat-suppressing region 90, as illustrated in Fig. 8 and Fig. 9, the convex portions 31, which are swollen toward a top side away from the second sheet layer 72, are provided in the first sheet layer 71 at intervals in the width direction WD and in the front-back direction LD in a staggered arrangement, and in addition, bonded portions 80 at which the first sheet layer 71 and the second sheet layer 72 are bonded to each other are formed between the convex portions 31 adjacent to each other in the width direction WD and between the convex portions 31 adjacent to each other in the front-back direction LD. A minimum width 32x of gap portions 32, each of which is disposed between the convex portions 31 adjacent to each other in the width direction WD is 0.1 to 0.9 times a maximum width 31c of the convex portions 31 located at both front and back sides of the gap portion 32. Further, at least that portion of the second sheet layer 72 which is corresponding to a whole of the pleat-suppressing region 90 is bonded to the elastic film 70.

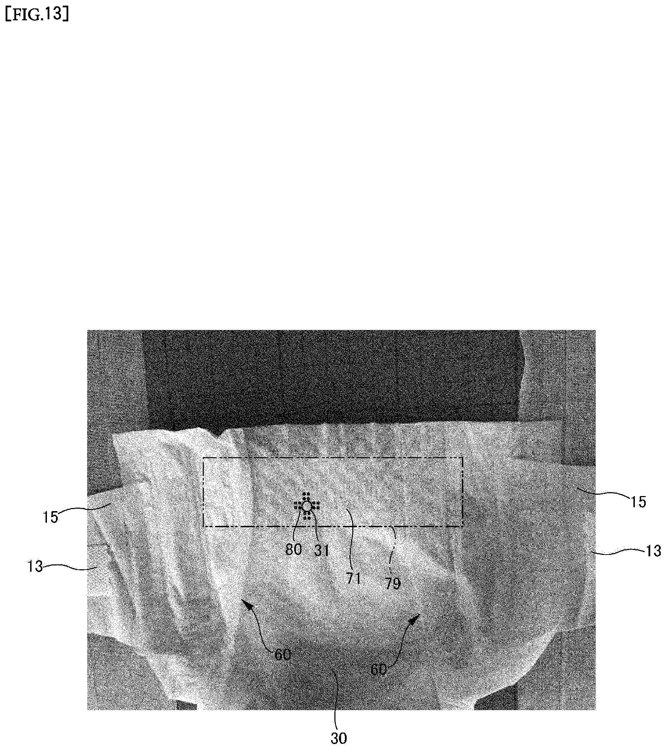

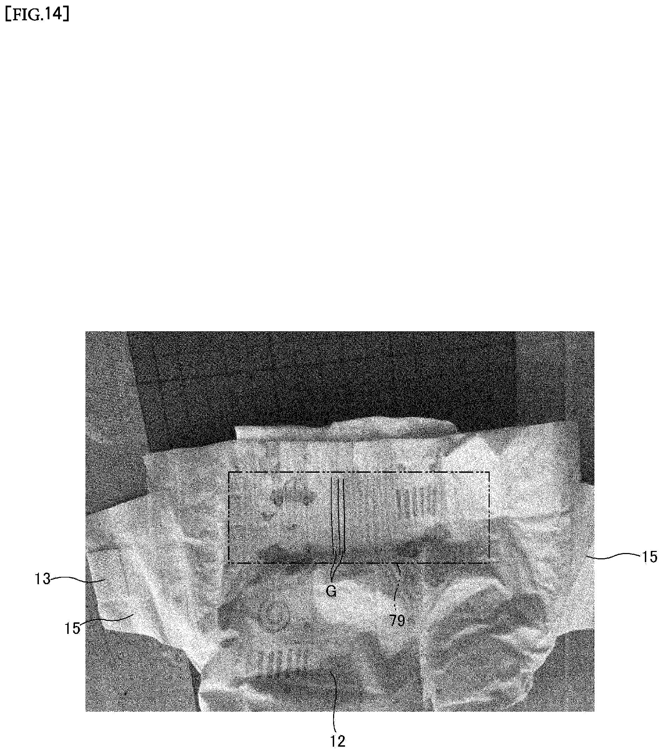

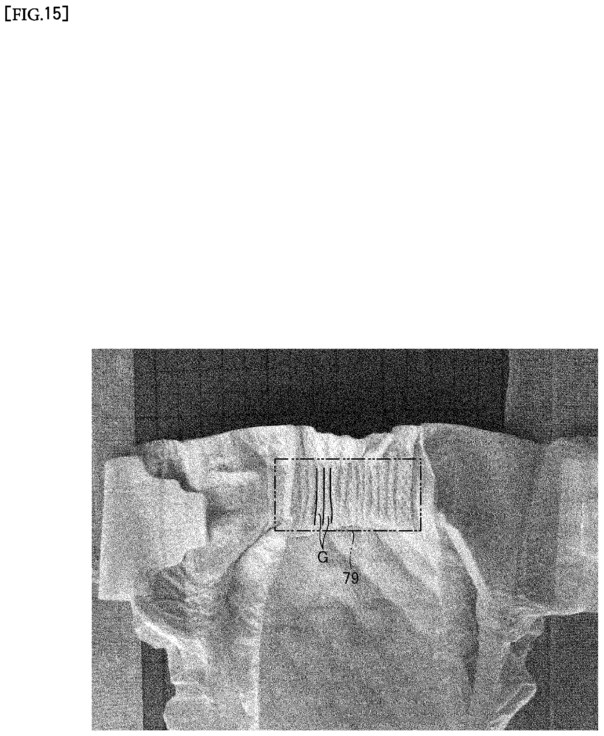

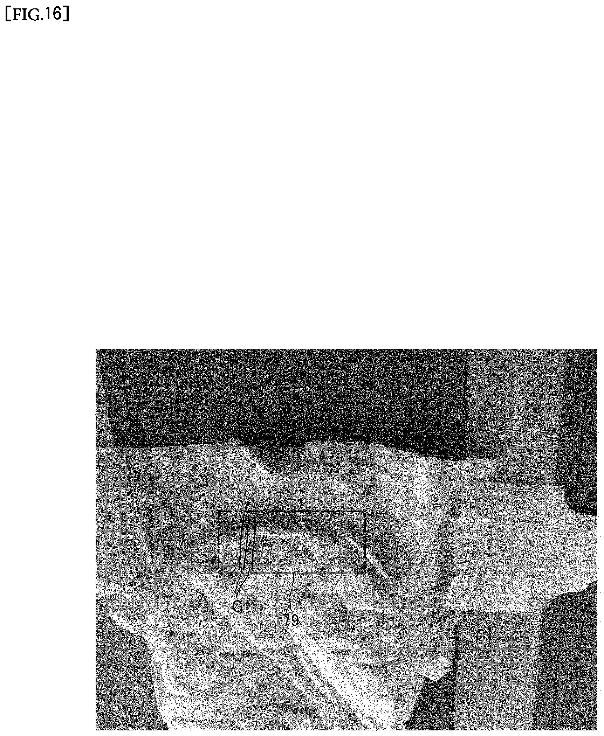

In such a waist end stretchable region 79, as known from a photograph (Fig. 13) of a top surface and a photograph (Fig. 14) of an underside surface of a sample in a natural length state adopting a structure illustrated in Fig. 1 to Fig. 9, pleats G are formed on an underside surface of the waist end stretchable region 79 while little or no pleats extending linearly in the front-back direction LD are formed on a top surface of the pleat-suppressing region 90. More closely, when the waist end stretchable region 79 stretches and contracts, in the pleat-suppressing region 90, although widths of the convex portions 31 and those of the gap portions 32 in the first sheet layer 71 also stretch and contract, little or no pleats are formed since a whole of the first sheet layer 71 does not deform wavily unlike a conventional wearing article. That is, since the convex portions 31, which are swollen toward the top side away from the second sheet layer 72, are previously provided in the first sheet layer 71 at intervals in the staggered arrangement, to a certain level of stretching and contracting, the whole of the first sheet layer 71 can stretch and contract in the width direction WD with little or no wavy deformation. As a result, even in the natural length state, little or no pleats are formed on the top surface of the pleat-suppressing region 90. On other hand, in a conventional diaper, as known from a photograph (Fig. 15) of a top surface and a photograph (Fig. 16) of an underside surface of a commercially available product in a natural length state, pleats are clearly formed on a top surface of the waist end stretchable region 79.

Meanwhile, a situation in which the convex portions 31 are previously formed in the first sheet layer 71 is, with regard to a possibility of leakage through portions between the convex portions 31, similar to a situation in which pleats are previously formed thereon. However, in the present invention, the convex portions 31 are provided in the staggered arrangement and the minimum width 32x of the gap portions 32, each of which is disposed between the convex portions 31 adjacent to each other in the width direction WD, is 0.1 to 0.9 times the maximum width 31c of the convex portions 31 located at both front and back sides of the gap portion 32. Accordingly, the portions between the convex portions 31 are not continuous linearly along the front-back direction LD but continuous in a net-like (wavelike) way. In this way, excrement cannot move backward without colliding with the convex portions 31, and the convex portions 31 become barriers to the movement of the excrement. Thus, prevention of the leakage is attained. On the other hand, the convex portions 31 provided in the staggered arrangement decrease area of contact between the first sheet layer 71 and wearer's skin, and in addition to this, the concave portions between the convex portions improve a breathability in a direction along a top surface of the first sheet layer 71. Incidentally, based on this viewpoint, the minimum width 32x of the gap portions 32, each of which is located between the convex portions 31 adjacent to each other in the width direction WD, is more preferably 0.5 to 0.9 times, and particularly preferably 0.7 to 0.9 of the maximum width 31c of the convex portions 31 located at both front and back sides of the gap portion 32.

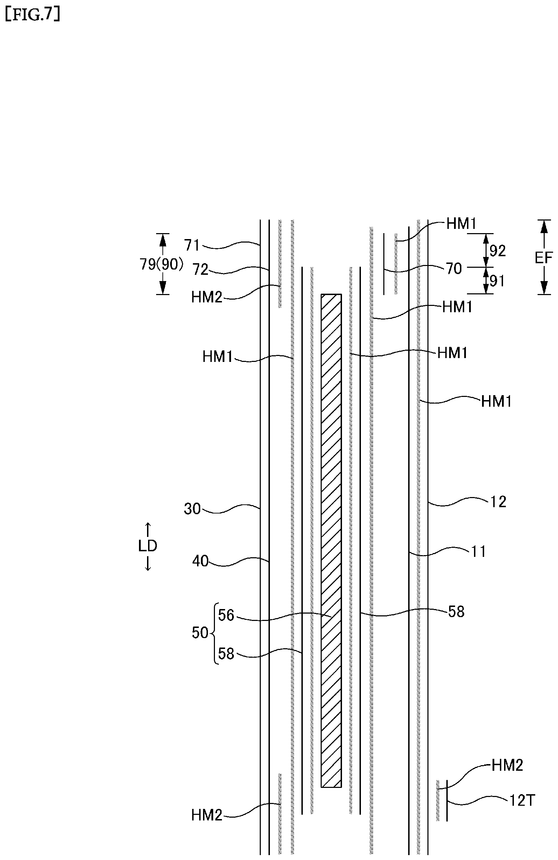

As for the pleat-suppressing region 90, it is necessary that at least that portion of the second sheet layer 72 which is corresponding to the whole of the pleat-suppressing region 90 is bonded to the elastic film 70. As for bonding between the second sheet layer 72 and the elastic film 70, direct bonding of a whole of the second sheet layer 72 and a whole of the elastic film 70 may be performed to each other, or indirect bonding thereof may be performed to each other through another layer. Further, as in the illustrated example (in particular, see Fig. 7), at a site, direct bonding of the second sheet layer 72 and the elastic film 70 may be performed, and at another site, indirect bonding thereof may be performed through another layer. As stated above, that portion of the second sheet layer 72 which is corresponding to the whole of the pleat-suppressing region 90 is bonded to the elastic film 70. This means that, when the waist end stretchable region 79 contracts, the second sheet layer 72 and the elastic film 70 are bonded to each other almost continuously so as not to generate a space therebetween. Accordingly, for example, the elastic film 70 and the second sheet layer 72 can be bonded to each other using interlayer adhesion provided entirely therebetween through a hot melt adhesive HM1 applied in a fine reticular pattern (also referred to as a fiber pattern) and a hot melt adhesive HM2 applied in a continuous plane. In a microscopic sense, in bonding portions by the hot melt adhesive HM1 applied in the fine reticular pattern, air pervious passages or liquid pervious passages in which any hot melt adhesive is not present are dotted. However, when the waist end stretchable region 79 contracts, such a space is not generated between the second sheet layer 72 and the elastic film 70. Note that, in order to apply the hot melt adhesive in the fine reticular pattern, spray application into a spiral shape, a Z shape, or the like can be used. On the other hand, in order to apply the hot melt adhesive in the continuous plane, slot application or the like can be used.

More specifically, the pleat-suppressing region 90 in the illustrated example includes a first region 91 having, between the first sheet layer 71 and the exterior nonwoven sheet 12, only the second sheet layer 72, the wrapping sheet 58, the elastic film 70, the liquid impervious sheet 11 and hot melt adhesives HM1, HM2, by which the first sheet layer 71, the second sheet layer 72, the wrapping sheet 58, the elastic film 70, the liquid impervious sheet 11 and the exterior nonwoven sheet 12 are bonded to each other, and a second region 92 having, between the first sheet layer 71 and the exterior nonwoven sheet 12, only the second sheet layer 72, the elastic film 70, the liquid impervious sheet 11 and the hot melt adhesives HM1, HM2, by which the first sheet layer 71, the second sheet layer 72, the elastic film 70, the liquid impervious sheet 11 and the exterior nonwoven sheet 12 are bonded to each other (that is, the second region 92 does not include the wrapping sheet 58). The elastic film 70 extends from between the liquid impervious sheet 11 and that portion of the wrapping sheet 58 which is located on the underside level with respect to the absorber 56 in the first region 91, to between the second sheet layer 72 and the liquid impervious sheet 11 in the second region 92.

In the pleat-suppressing region 90 having such a stacked structure, a whole of an underside surface of the second sheet layer 72 can be bonded, in the first region 91, to a top surface of that portion of the wrapping sheet 58 which is located on a top side and in the second region 92, to a top surface of the elastic film 70, through the hot melt adhesive HM1 applied in the fine reticular pattern and the hot melt adhesive HM2 applied in the continuous plane, in the first region 91, facing surfaces of that portion of the wrapping sheet 58 which is located on the top side, and that portion of the wrapping sheet 58 which is located on an underside, can be bonded entirely to each other, through the hot melt adhesive HM1 applied in the fine reticular pattern, and in the first region 91, an underside surface of that portion of the wrapping sheet 58 which is located on the underside and a top surface of the elastic film 70 can be bonded entirely to each other, through the hot melt adhesive HM1 applied in the fine reticular pattern.

Incidentally, as in the illustrated example, in the pleat-suppressing region 90, stacked sheets other than the elastic film 70 are preferably few in number, from a view point of reducing producing costs. However, only by decreasing the stacked number of sheets, stiffness of the pleat-suppressing region 90 becomes lower and this region 90 would be likely to bend at a boundary between the first region 91 and the second region 92 and at a tip end of the end flap part EF in the dorsal side part B. On the other hand, in the present invention, the convex portions 31 are formed in the first sheet layer 71, and in addition to this, the bonded portions 80, at which the first sheet layer 71 and the second sheet layer 72 are bonded to each other, are formed, as explained above. In this way, the stiffness of the pleat-suppressing region 90 becomes higher comparing with a case in which there is no convex portion and no bonded portion, such that the pleat-suppressing region 90 is unlikely to bend at the boundary between the first region 91 and the second region 92 and at the tip end of the end flap part EF in the dorsal side part B.

In the pleat-suppressing region 90, too large stretch rate makes it easy for pleats to be formed. Thus, the stretch rate of the waist end stretchable region 79 in a spread state is preferably 130 to 1700, more preferably 130 to 1500. Incidentally, in the sample illustrated in Fig. 13 and Fig. 14, the stretch rate of the waist end stretchable region 79 in a spread state is 1400.

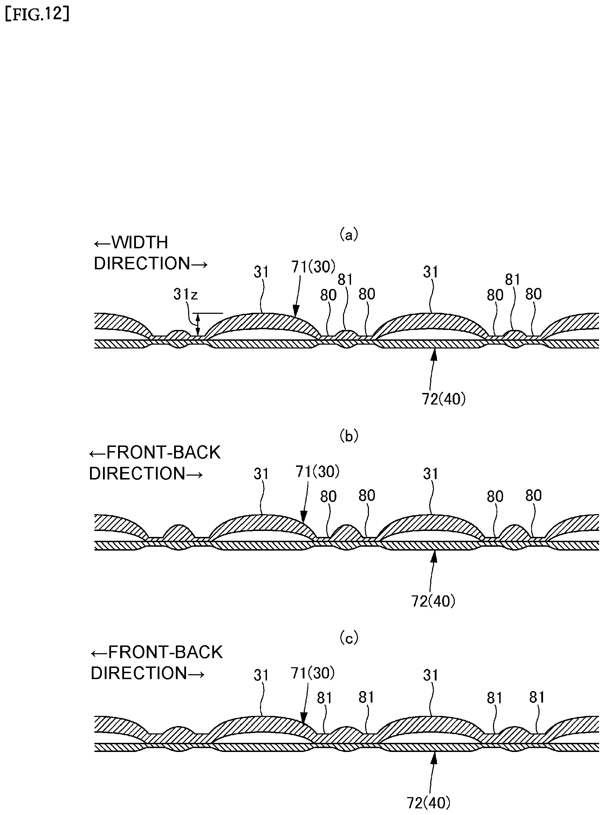

It is enough that the convex portions 31 formed in the first sheet layer 71 are swollen away from the second sheet layer 72 as illustrated in Fig. 12. Such convex portions 31 can be formed by extruding the first sheet layer 71 from an underside to a top side thereof by means of embossing. Each of the convex portions 31 has preferably a circular dome shape, but can have an appropriate shape such as an elliptical dome shape and a polygonal dome shape.

A dimension and the like of the convex portion 31 can be determined as appropriate. However, as illustrated in Fig. 9 to Fig. 11, a maximum length 31m (a dimension in the front-back direction LD) of each of the convex portions 31 is smaller than a center interval 80y of a bonded portion 80 (discussed later) located at one side and a bonded portion 80 located at the other side in the front-back direction LD of the convex portion 31, a lower limit of the maximum length 31m is preferably about 0.9 times the center interval 80y, and the maximum length 31m of a diaper for babies and infants is preferably about 3 to 9 mm. Similarly, a maximum width 31c (a dimension in the width direction WD) of each of the convex portions 31 is smaller than a center interval 80x of a bonded portion 80 located at one side and a bonded portion 80 located at the other side in the width direction WD of the convex portion 31, a lower limit of the maximum width 31c is preferably about 0.9 times the center interval 80x, and the maximum width 31c of a diaper for babies and infants is preferably about 3 to 9 mm. A minimum length 32y in the front-back direction LD of each of the gap portions 32 located between the convex portions 31 adjacent to each other in the width direction WD can be about 0.8 to 1.2 times the minimum width 32x.

A disposition interval of the convex portions 31 can be determined as appropriate. As an example, it is possible that a center interval 31x in the width direction WD of the convex portions 31 adjacent to each other in the width direction WD in respective rows in each of which the convex portions 31 are arranged in the front-back direction LD can be about 3 to 10 mm, and a center interval 31y in the front-back direction LD of the convex portions 31 adjacent to each other in the front-back direction LD in respective rows in each of which the convex portions 31 are arranged in the width direction WD can be about 3 to 10 mm. Particularly in the pleat-suppressing region 90, rows each of which includes 5 to 15 convex portions 31 arranged in the front-back direction LD are preferably provided, from viewpoints of an effect for suppressing the pleats and an effect for preventing the leakage.

It is preferable that a height 31z of each of the convex portions 31 (see Fig. 12) is preferably 0.20 to 0.65 times a thickness of the absorber 56. In this way, since the height 31z of each of the convex portions 31 of the first sheet layer 71 is enough with respect to the thickness of the absorber 56 as a reference, a cushioning property (recoverability from a compressed situation in a thickness direction) of the end flap part EF in the dorsal side part is improved. As a result, a top surface of the end flap part EF in the dorsal side part becomes likely to be brought into contact with the wearer's body surface. Note that the height 31z of each of the convex portions 31 means an apparent height (in a state where an external force is not applied), which can be measured by a digital microscope VHS-1000 manufactured by KEYENCE CORPORATION.

For forming the bonded portion 80 at which the first sheet layer 71 and the second sheet layer 72 are bonded to each other, these sheet layers 71 and 72 are bonded to each other by welding such as heat sealing or ultrasonic sealing, or by the hot melt adhesive.

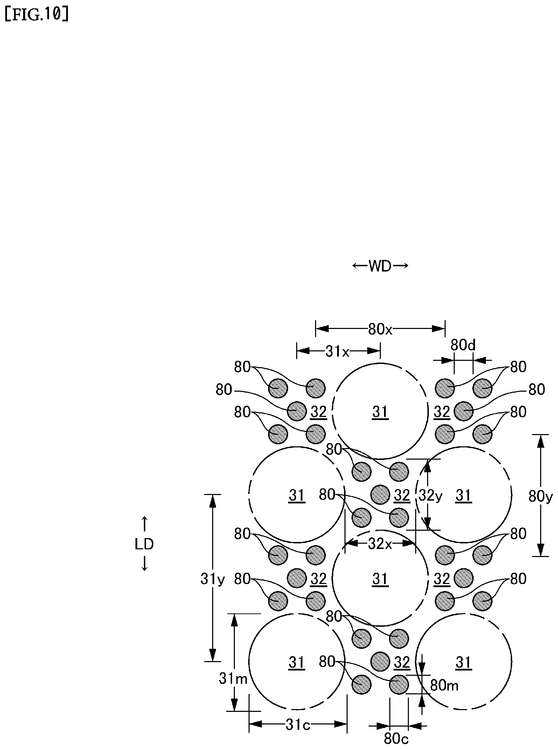

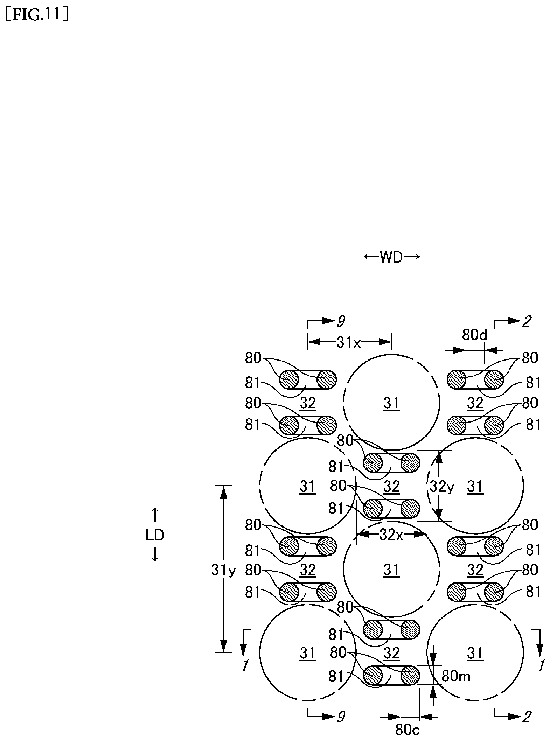

As long as the bonded portion 80 is provided between the convex portions 31 adjacent to each other in the width direction WD and in the front-back direction LD, a plurality of bonded portions 80 may be provided per one portion between the convex portions 31, or one bonded portion 80 may be provided per one portion between the convex portions 31. Further, in a case where the plurality of bonded portions 80 are provided per one portion between the convex portions 31, the bonded portions 80 may be arranged in a single row, in a matrix arrangement as in the examples illustrated in Fig. 9 and Fig. 11, or in a staggered arrangement as in the example illustrated in Fig. 10. A shape of the bonded portion 80 can be determined as appropriate, and can be set to any shape such as an elliptical shape, a polygonal shape, a star shape, a cloud shape, a gourd shape and the like, in addition to a circular shape as in the illustrated examples.

A dimension of the bonded portion 80 can be determined as appropriate. However, a maximum length 80m (a dimension in the front-back direction LD) of each of the bonded portions 80 is preferably about 0.1 to 0.4 times the center interval 31y in the front-back direction LD of the convex portions 31 adjacent to each other in the front-back direction LD in the respective rows in each of which the convex portions 31 are arranged in the width direction WD (for example, the maximum length 80m is 0.5 to 3 mm in a diaper for babies and infants). Further, the maximum width 80c (a dimension in the width direction WD) of each of the bonded portions 80 is preferably about 0.1 to 0.4 times the center interval 31x in the width direction WD of the convex portions 31 adjacent to each other in the width direction WD in the respective rows in each of which the convex portions 31 are arranged in the front-back direction LD (for example, the maximum width 80c is 0.5 to 3 mm in a diaper for babies and infants).

As in the example illustrated in Fig. 11, in the gap portions, each of which is between the bonded portions 80 adjacent to each other in the width direction WD, the first sheet layer 71 and the second sheet layer 72 are not bonded to each other, and the first sheet layer 71 is compressed more deeply than portions located at both sides thereof in the front-back direction LD to form compressed portions 81. In the compressed portions 81, as long as the first sheet layer 71 is compressed, the second sheet layer 72 may be compressed integrally together with the first sheet layer 71 or may not be compressed.

As long as the convex portions 31 and the bonded portions 80 are provided over the whole of the pleat-suppressing region 90 (a portion indicated by a hatch pattern in Fig. 8), a range in which the convex portions 31 and the bonded portions 80 having the same configurations and arrangements are provided may extend outward beyond the pleat-suppressing region 90. For example, as in the illustrated example, in a case where the top sheet 30 forming the first sheet layer 71 and the intermediate sheet 40 forming the second sheet layer 72 extend outward beyond the pleat-suppressing region 90 in the front-back direction LD (in the illustrated example, they extend over the entire length of the product in the front-back direction LD), the convex portions 31 and the bonded portions 80 may be provided over the entire length in the front-back direction of a range in which the top sheet 30 and the intermediate sheet 40 are overlapped.

A region in which the convex portions 31 and the bonded portions 80 are arranged may extend over a wider range than that of the waist end stretchable region 79 so as to include it, or may extend over the same range as that of the waist end stretchable region 79. Alternatively, the convex portions 31 and the bonded portions 80 may be provided only in a part of the waist end stretchable region 79 (for example, only in an intermediate portion of the waist end stretchable region 79 excluding both side portions thereof in the width direction WD).

### (Nonwoven Fabric)

As a nonwoven fabric in the above description, a known nonwoven fabric can be appropriately used according to a site or a purpose. Examples of a constituent fiber of the nonwoven fabric include, but are not limited to, a synthetic fiber such as a polyolefin-based fiber including polyethylene and polypropylene, a polyester-based fiber, or a polyamide-based fiber (including a composite fiber such as core-sheath in addition to a single component fiber), a regenerated fiber such as rayon or cupra, and a natural fiber such as cotton. These fibers can be mixed and used. In order to enhance flexibility of the nonwoven fabric, it is preferable to use a crimped fiber as the constituent fiber. In addition, the constituent fiber of the nonwoven fabric may be a hydrophilic fiber (including a fiber that has become hydrophilic by a hydrophilizing agent), a hydrophobic fiber, or a water-repellent fiber (including a fiber that has become water-repellent by a water repellent agent). In addition, the nonwoven fabric is generally classified into a short fiber nonwoven fabric, a long fiber nonwoven fabric, a spunbonded nonwoven fabric, a meltblown nonwoven fabric, a spunlace nonwoven fabric, a thermal bond (air through) nonwoven fabric, a needle punch nonwoven fabric, a point bond nonwoven fabric, a laminated nonwoven fabric (an SMS nonwoven fabric, an SMMS nonwoven fabric, or the like having a meltblown layer sandwiched between spunbond layers), and the like depending on a fiber length, a sheet forming method, a fiber bonding method, and a stacked structure, and any of these nonwoven fabrics can be used.

### <Description of Terms Used in Specification>

The following terms used in the specification should be understood to have the meanings defined below unless otherwise specified in the specification.

- "Front-back direction" means a direction (longitudinal direction) indicated by a reference character LD in the drawing, "width direction" means a direction (left-right direction) indicated by a reference character WD in the drawing, and the front-back direction and the width direction are orthogonal to each other.
- "MD" and "CD" mean the flow direction (MD) in a manufacturing equipment and the lateral direction (CD) orthogonal to the flow direction, and either one is the front-back direction and the other is the width direction depending on each member of a product. The MD of a nonwoven fabric is the direction of fiber orientation of the nonwoven fabric. The fiber orientation is a direction along which a fiber of a nonwoven fabric runs and determined by, for example, a measurement method in accordance with the fiber orientation test method based on the zero distance tensile strength of TAPPI standard method T481 or a simple measurement method for determining the fiber orientation direction from the tensile strength ratio of the front-back direction and the width direction.
- "Top side" means a side closer to wearer's skin when an article is worn. "Underside" means a side far from wearer's skin when an article is worn.
- "Top surface" means a surface of a member closer to wearer's skin when an article is worn. "Underside surface" means a surface far from wearer's skin when an article is worn.
- "Stretch rate" means a value obtained when a natural length is set to 100%. For example, the stretch rate of 200% has the same meaning as the elongation ratio of 2.
- "Gel strength" is measured as follows. 1.0 g of super absorbent polymer is added to 49.0 g of artificial urine (prepared by mixing 2wt% of urea, 0.8wt% of sodium chloride, 0.03wt% of calcium chloride dihydrate, 0.08wt% of magnesium sulfate heptahydrate, and 97.09wt% of ion exchanged water), and the resulting mixture is agitated with a stirrer. The resulting gel is left in a thermohygrostat at 40°C × 60%RH for three hours and then cooled to room temperature. The gel strength of the gel is measured with a curdmeter (Curdmeter-MAX ME-500 manufactured by I. Techno Engineering Co., Ltd.).
- "Basis weight" is measured as follows. After preliminary drying of a sample or a test piece, the sample or the test piece is left in a test room or a test device under normal conditions (an ambient temperature of 23 ± 1°C and with a relative humidity of 50 ± 2% at the testing site) until the weight of sample or test piece reaches constant mass. Preliminary drying is to achieve the constant mass of the sample or test piece under an environment having a temperature of 100°C. For fibers having a standard moisture regain of 0.0%, preliminary drying may be omitted. The test piece having the constant mass is cut with a cutting template having the size of 100 mm × 100 mm into samples having the size of 100 mm × 100 mm. The weight of the sample is measured. The measured weight is multiplied by 100 to determine the weight per one square meter, which is defined as the basis weight.
- The "thickness" of a thick member such as the absorber 56, the absorbent element 50, or the like is measured using a thickness measuring instrument of Ozaki Mfg. Co., Ltd. (Peacock, Dial Thickness Gauge, Model G-30 (measurement range of 0 to 10 mm, dimension and shape of measurement surface : a circular contact point with a diameter of 60 mm, measuring force of about 1.7 N) by horizontally placing the sample and the thickness measuring device. The "thickness" other than the above thickness of a thin member such as a nonwoven fabric is automatically measured under the condition of load: 0.098 N/cm² and pressure area: 2 cm² using an automatic thickness meter (KES-G5 handy compression measurement program). The thickness of a perforated sheet such as a perforated nonwoven fabric is measured at a portion other than the holes or projections around the holes.
- Water absorption capacity is measured in accordance with JIS K7223-1996 "Testing method for water absorption capacity of super absorbent polymers".
- Water absorption rate is defined as "time that elapses before the end point" measured with 2g of super absorbent polymers and 50g of normal saline solution in accordance with JIS K7224-1996 "Testing method for water absorption rate of super absorbent polymers".
- The fiber orientation of a nonwoven fabric is a direction along which a fiber of the nonwoven fabric runs and determined by, for example, a measurement method in accordance with the fiber orientation test method based on the zero distance tensile strength of TAPPI standard method T481 or a simple measurement method for determining the fiber orientation direction from the tensile strength ratio of the front-back direction and the width direction.
- "Spread state" means a state where an article is spread flat without contraction (including any contraction such as contraction by an elastic member) or slackness.
- The dimension of each component means a dimension in a spread state, not in a natural length state, unless otherwise specified.

The tests and measurements are carried out in a laboratory or apparatus under normal conditions (a temperature of 23 ± 1°C and a relative humidity of 50 ± 2% at the testing site), unless the environmental condition for the tests and measurements are otherwise specified.

### Industrial Applicability

The present invention is applicable to connecting-type disposable wearing articles such as tape-type disposable diapers as in the above examples.

### Reference Signs List

- 11:: Liquid impervious sheet
- 12 :: Exterior nonwoven sheet
- 12T:: Target sheet
- 13 :: Connecting tape
- 13A :: Connecting portion
- 13B :: Main unit portion
- 13C :: Base end portion
- 30 :: Top sheet
- 31 :: Convex portion
- 32 :: Gap portion
- 40 :: Intermediate sheet
- 50 :: Absorbent element
- 56 :: Absorber
- 58 :: Wrapping sheet
- 60 :: Rising gather
- 62 :: Gather sheet
- 63 :: Gather elastic member
- 65 :: Root portion
- 66 :: Main portion
- 67b :: Back fallen portion
- 67f :: Front fallen portion
- 68 :: Rising portion
- 70 :: Elastic film
- 71 :: First sheet layer
- 72 :: Second sheet layer
- 79 :: Waist end stretchable region
- 80 :: Bonded portion
- 90 :: Pleat-suppressing region
- B :: Dorsal side part
- EF :: End flap part
- F :: Ventral side part
- LD :: Front-back direction
- SF :: Side flap part
- WD :: Width direction
- WP :: Wing part

## Claims

1. A connecting-type disposable wearing article comprising:
a ventral side part extending forward from a center in a front-back direction, and a dorsal side part extending backward from the center in the front-back direction,
an absorber incorporated within a range from the ventral side part to the dorsal side part,
connecting portions, which are provided in both side portions of the dorsal side part and configured to be detachably connected to an external surface of the ventral side part,
an end flap part, which extends backward so as to be located at a back side of the absorber,
wherein the end flap part includes
a first sheet layer forming a skin-contact surface,
a second sheet layer bonded to an underside surface of the first sheet layer, and
an elastic film stacked in an underside of the second sheet layer,
the first sheet layer and the second sheet layer are made of nonwoven fabrics respectively,
a region including the elastic film has a waist end stretchable region, which can contract in a width direction and stretch in the width direction together with the elastic film,
the waist end stretchable region has a pleat-suppressing region, in which the first sheet layer, the second sheet layer and the elastic film are stacked,
at least in the pleat-suppressing region, convex portions, which are swollen toward a top side away from the second sheet layer, are provided in the first sheet layer at intervals in the front-back direction and in the width direction in a staggered arrangement, and the first sheet layer and the second sheet layer are bonded to each other at bonded portions, each of which is formed between the convex portions adjacent to each other in the width direction and between the convex portions adjacent to each other in the front-back direction,
a minimum width of gap portions, each of which is located between the convex portions adjacent to each other in the width direction, is 0.1 to 0.9 times a maximum width of the convex portions, which are located at both front and back sides of the gap portion, and
at least a portion of the second sheet layer which is corresponding to a whole of the pleat-suppressing region is bonded to the elastic film.

2. The connecting-type disposable wearing article according to claim 1,
wherein a stretch rate of the waist end stretchable region in a spread state is 130 to 1700.

3. The connecting-type disposable wearing article according to claim 1 or 2,
wherein, a maximum width of each of the convex portions is 3 to 9 mm, and
in the pleat-suppressing region, rows, each of which includes 5 to 15 convex portions arranged in the front-back direction, are provided.

4. The connecting-type disposable wearing article according to any one of claims 1 to 3,
wherein a height of each of the convex portions is 0.20 to 0.65 times a thickness of the absorber.

5. The connecting-type disposable wearing article according to any one of claims 1 to 4,
wherein the first sheet layer is made of a short fiber nonwoven fabric having a fineness of 1 to 3 dtex, a basis weight of 18 to 30 g/m², and a thickness of 0.3 to 1.4 mm, and
the second sheet layer is made of a short fiber nonwoven fabric having a fineness of 2 to 6 dtex, a basis weight of 17 to 30 g/m², and a thickness of 0.2 to 4 mm.

6. The connecting-type disposable wearing article according to any one of claims 1 to 5, further comprising:
a wrapping sheet wrapping the absorber;
a liquid impervious sheet disposed in an underside of that portion of the wrapping sheet which is located on an underside of the absorber; and
an exterior nonwoven sheet covering an underside surface of the liquid impervious sheet to constitute an external surface of the wearing article,
wherein the wrapping sheet extends to a middle of the end flap part in the front-back direction,
the first sheet layer, the second sheet layer, the liquid impervious sheet and the exterior nonwoven sheet extend over an entire length of the wearing article,
the pleat-suppressing region includes
a first region having, between the first sheet layer and the exterior nonwoven sheet, only the second sheet layer, the wrapping sheet, the elastic film, the liquid impervious sheet and a hot melt adhesive for bonding them, and
a second region having, between the first sheet layer and the exterior nonwoven sheet, only the second sheet layer, the elastic film, the liquid impervious sheet and the hot melt adhesive for bonding them, and
the elastic film extends from between the liquid impervious sheet and the portion of the wrapping sheet which is located on the underside level with respect to the absorber in the first region to between the second sheet layer and the liquid impervious sheet in the second region.
